# EUROPEAN PATENT SPECIFICATION

(11) **EP 3 788 173 B1**
(45) Date of publication and mention of the grant of the patent: **29.11.2023**
(21) Application number: 19724365.2
(22) Date of filing: 03.05.2019
(51) Int. Cl.: C12Q 1/6886

(54) **SURROGATE MARKER AND METHOD FOR TUMOR MUTATION BURDEN MEASUREMENT**
SURROGATMARKER UND VERFAHREN ZUR MESSUNG DER TUMORMUTATIONSBELASTUNG
MARQUEUR DE SUBSTITUTION ET PROCÉDÉ DE MESURE DE CHARGE DE MUTATION DE TUMEUR

(30) Priority: 03.05.2018 US 201862666329 P
(43) Date of publication of application: 10.03.2021
(73) Proprietor: F. Hoffmann-La Roche AG, 4070 Basel (CH); Roche Diagnostics GmbH, 68305 Mannheim (DE)
(72) Inventor: BALASUBRAMANYAM, Aarthi, Pleasanton, California 94588 (US); JU, Christine, Pleasanton, California 84588 (US); MA, Xiaoju, Pleasanton, California 94588 (US); MULEY, Thomas, 69126 Heidelberg (DE); HERTH, Felix, 69126 Heidelberg (DE); TIKOO, Nalin, Pleasanton, California 94588 (US); WEHNL, Birgit, 82377 Penzberg (DE); XI, Liu, Pleasanton, California 94588 (US); YAUNG, Stephanie J., Pleasanton, California 94588 (US); PATI, Amrita, Pleasanton, California 94588 (US)
(74) Representative: Hildebrandt, Martin K. E.
(86) International application number: PCT/EP2019/061328
(87) International publication number: WO 2019/211418

(56) References cited:
- WO-A1-2015/181213
- WO-A1-2017/151524
- J JIANG ET AL: "Longitudinal Mutation Monitoring in Plasma by Deep Sequencing as a Potential Predictor of Disease Progression in NSCLC", JOURNAL OF THORACIC ONCOLOGY, vol. 12, no. 11S2, 1 November 2017 (2017-11-01), pages S1772-S1773, XP055611254, DOI: https://doi.org/10.1016/j.jtho.2017.09.386 & Anonymous: "AVENIO ctDNA Surveillance Kit", , 1 September 2018 (2018-09-01), XP055610861, Retrieved from the Internet: URL:https://sequencing.roche.com/content/d am/rochesequence/worldwide/resources/broch ure-avenio-ctdna-surveillance-kit-SEQ10004 8.pdf [retrieved on 2019-08-05]
- Luís Felipe Campesato ET AL: "Comprehensive cancer-gene panels can be used to estimate mutational load and predict clinical benefit to PD-1 blockade in clinical practice", Oncotarget, 27 October 2015 (2015-10-27), XP055532035, DOI: 10.18632/oncotarget.5950 Retrieved from the Internet: URL:https://www.ncbi.nlm.nih.gov/pmc/artic les/PMC4741447/pdf/oncotarget-06-34221.pdf & Luis Felipe Campesato: "Comprehensive cancer-gene panels can be used to estimate mutational load and predict clinical benefit to PD-1 blockade in clinical practice - Supplementary Material", , 1 October 2015 (2015-10-01), XP055611165, Retrieved from the Internet: URL:http://www.oncotarget.com/index.php?jo urnal=oncotarget&page=rt&op=suppFiles&path []=5950&path[]=14285 [retrieved on 2019-08-06]
- GUAN-YI LYU ET AL: "Mutation load estimation model as a predictor of the response to cancer immunotherapy", NPJ GENOMIC MEDICINE, vol. 3, no. 1, 30 April 2018 (2018-04-30), XP055611226, DOI: 10.1038/s41525-018-0051-x
- YUKI OWADA-OZAKI ET AL: "Prognostic Impact of Tumor Mutation Burden in Patients With Completely Resected Non-Small Cell Lung Cancer: Brief Report", MOLECULAR THERAPY, vol. 13, no. 8, 11 April 2018 (2018-04-11) , pages 1217-1221, XP055610807, ISSN: 1556-0864, DOI: 10.1016/j.jtho.2018.04.003

## Description

### FIELD OF THE INVENTION

The invention relates to the field of oncology. More specifically, the invention relates to the field of nucleic acid-based testing of cancer patients.

### BACKGROUND OF THE INVENTION

Tumor cell are known to accumulate somatic mutations during tumor development and progression. Tumor Mutation Burden (TMB) is defined as a number of mutations in a tumor sample from a patient. TMB is an established biomarker for chemotherapies and cancer immunotherapies for an array of solid metastatic malignancies. TMB is often measured by whole exome sequencing (WES) or by sequencing of multi-megabase panels. These established methods are not only costly but also require tissue specimens. Some examples are disclosed in WO 2015/181213 A1, WO 2017/151524 A1, JIANG ET AL (JOURNAL OF THORACIC ONCOLOGY, vol. 12, no. 11S2, 1 November 2017 pages S1772-S1773), Campesato ET AL (Oncotarget, 27 October 2015, p. 5950), LYU ET AL (NPJ GENOMIC MEDICINE, vol.3, no.1, 30 April 2018 ), and OWADA-OZAKI ET AL MOLECULAR THERAPY, vol. 13, no. 8, p. 1217-1221). For some of the late stage cancer patients, tissue biopsy is inaccessible. There is a need for a reliable and practical method to assess a patient's mutation status and TMB in order to optimally guide therapy.

### SUMMARY OF THE INVENTION

In some embodiments, the invention is a method for determining mutation burden in a lung cancer patient comprising the steps of: isolating nucleic acids from a cell-free blood sample obtained from the patient; in the isolated nucleic acid, determining the sequence of at least a portion of each of the biomarkers listed in Table 1; comparing the sequence to the reference sequence and identifying mutations; determining mutation burden as the ratio of the number of mutations to the number of bases of nucleic acid sequenced. The patient may be a cancer patient diagnosed with one of carcinoma, sarcoma, myeloma, leukemia or lymphoma. In some embodiments, only non-synonymous mutations are used in determining the mutation burden. In some embodiments, mutations in cancer driver genes are excluded from determining the mutation burden.

In some embodiments, the invention is a method of determining prognosis for a lung cancer patient comprising the steps of: isolating nucleic acids from a cell-free blood sample obtained from the patient; in the isolated nucleic acid, determining the sequence of at least a portion of each of the biomarkers listed in Table 1; comparing the sequence to the reference sequence and identifying mutations; determining mutation burden as a ratio of the number of mutations to the number of bases of nucleic acid sequenced; determining prognosis as poor if the mutation burden is high and determining prognosis as good if the mutation burden is low.

In some embodiments, the invention is a system for determining mutation burden in a patient, the system comprising a computer designed to implement an algorithm for detecting mutation burden in a sample from a cancer patient, wherein the algorithm analyses sequencing data on biomarkers from Table 1 and contains steps of mutation detection, error correction, determining a mutation burden as a ratio of the number of mutations identified to the number of bases of nucleic acid sequenced and determining whether the mutation burden in the sample falls above or below the bottom tertile of samples from the same cancer type.

### BRIEF DESCRIPTION OF THE DRAWINGS

Figure 1 is a graph showing correlation of a gene panel-based mutation counts with whole exome-based mutation counts derived from lung cancer samples.
Figure 2 shows progression free survival (PFS) and overall survival (OS) of patients classified by mutation burden based on a gene panel.

### DETAILED DESCRIPTION OF THE INVENITON

### Definitions

The following definitions are not limiting but merely aid in understanding this disclosure.

The term "PFS" is used herein to describe the time of Progression Free Survival for a patient.

The term "OS" is used herein to describe the time of Overall Survival for a patient.

The term "circulating tumor DNA (ctDNA)" is used herein to describe a portion of cell-free DNA found in human blood plasma or serum that originates from the tumor. Circulating tumor DNA is distinguished from non-tumor DNA by the mutations characteristic of the tumor. In the context of the present invention, detecting ctDNA means detecting mutated cell-free DNA.

The term "cancer driver gene" is used herein to describe one whose mutations and resulting aberrant activity increase net cell growth. Some examples of cancer driver genes include EGFR, KRAS, BRAF, ALK.

The term "biomarker" is used herein to describe a nucleotide sequence that contains information relevant to the biological or clinical phenomenon. For example, the information may be a mutation status of the sequence. The biomarker can be a gene (including coding sequence, regulatory sequence, intron or a splice site) or an intergenic region. The clinical phenomenon can be the presence of malignant cells, e.g., tumor cells in a patient's sample.

The term "whole genome sequencing of WGS" is used herein to describe sequencing of the entire genome of a cell or organism from which a cells or cells are derived. The term "whole exome sequencing of WES" is used herein to describe sequencing of all the exons present in all the genes of the genome. Both WGS and WES on human or other higher order vertebrate genomes are performed using massively parallel sequencing methods (next generation sequencing methods) capable of gathering and storing large amounts of sequence information.

This invention is intended to provide an alternative approach to the currently established TMB measurement. Given that tumors shed DNA into the circulation, we hypothesized that the mutation count in the circulating tumor DNA (ctDNA) may serve as an alternative to the tissue-based TMB measurement. However, detecting mutations in ctDNA requires much greater depth of sequencing than tissue-based sequencing, which requires us to use a smaller targeted sequencing panel that enriches the commonly mutated genes in solid tumors. To this end, we assessed mutation count using the AVENIO ctDNA Surveillance Kit, a targeted next-generation sequencing panel of 198 kilobases, on baseline plasma samples. We hypothesized that this small panel may serve as a surrogate for the multi-megabase sequencing or WES.

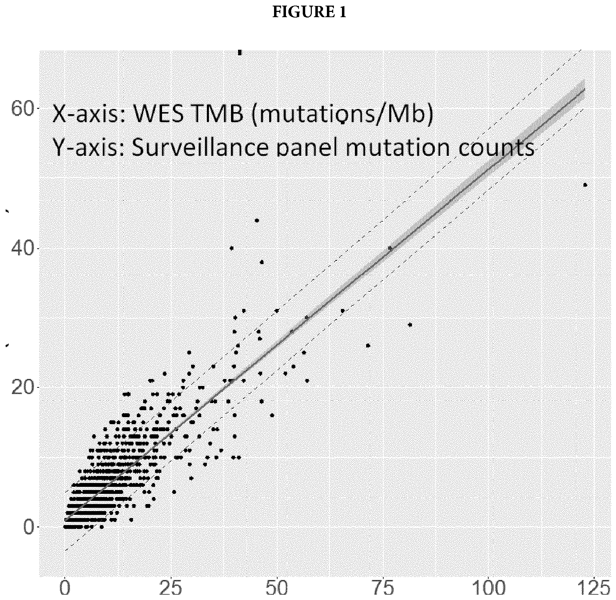

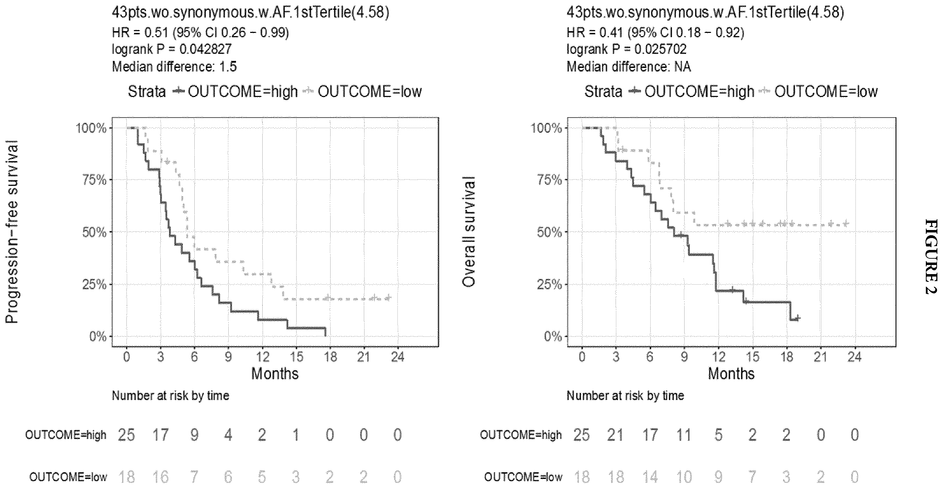

We took two independent approaches to demonstrate that the AVENIO ctDNA Surveillance Panel may serve as a surrogate for tumor tissue based TMB measurement. First, we undertook an *in silico* analysis of published whole exome sequence (WES) datasets to compare total mutation counts and mutation counts derived from *in silico* capture of mutations using the AVENIO Surveillance panel. As described in detail in Example 1, a correlation was obtained (Figure 1). Second, a set of patient-derived samples was analyzed using the AVENIO ctDNA Surveillance Panel. The patients were grouped by mutation burden (Example 2). Next, progression-free survival (PFS) and overall survival (OS) of the High and Low mutation groups were compared. A clear distinction between the groups was observed. (Figure 2).

Taken together, the results demonstrate the utility of the present invention. The invention comprises a novel approach of using a limited gene panel applied to cell-free tumor DNA to assess a total mutation burden (TMB) in a patient. The results demonstrate that analysis of a panel the size of one-fifth of a megabase is a surrogate for TMB traditionally assessed by sequencing an entire exome of the patient.

In some embodiments, the invention utilizes a blood sample from a patient.

The sample can include any fraction of blood, *e.g.,* serum or plasma, which contains cell-free DNA including circulating tumor DNA (cfDNA or ctDNA). In some embodiments, one or more sample is taken serially at various times during treatment, *e.g.,* before and after surgery or before, after and during a chemotherapy regimen, a chemoradiation therapy regimen or a regimen of adjuvant therapy. The blood sample can be collected by a suitable means that preserves the cell-free DNA therein, including collecting blood or blood plasma in a preservative medium.

The invention utilizes a biomarker panel, including a gene panel or a mutation panel or a somatic variant panel. The mutations may include single-nucleotide variations (SNVs), deletions and insertions (in-dels) that correspond to on-sense missense and frame-shift mutations if they occur in the coding regions of genes. Other types of mutations include gene fusions and translocations. The selection, size and content of such panels has been described *e.g.,* in U.S. Patent applications Ser. No. 14/774,518 and International app. No. PCT/US2015/049838 titled "Identification and Use of Circulating Tumor Markers." In some embodiments, the invention includes determining the sequence of the biomarkers in the panel, e.g., the genes listed in Table 1. In some embodiments, the entire sequence of a gene is determined. In other embodiments, the entire coding sequence of a gene is determined. In other embodiments, only the sequence of a portion of the gene known to undergo mutagenesis in cancer is determined. In yet other embodiments, the biomarker is not associated with a coding sequence but is associated with a regulatory sequence or a sequence of unknown function known to be mutated in human tumors.

In some embodiments, the invention utilizes a biomarker panel, such as AVENIO^{®} ctDNA Analysis Kit (Roche Sequencing Solutions, Inc., Pleasanton, Cal.) that is capable of analyzing the blood of patients after surgery to identify whether patients have circulating tumor DNA (ctDNA). In some embodiments, a panel that represents NCCN guideline recommended biomarkers for targeted therapies (17 genes) is used. In other embodiments, a broader panel further including therapy resistance markers (total 60 genes) is used. In yet another example, a broader panel further including cancer hotspot mutations (total 180 genes) is used (see the patent applications "Identification and Use of Circulating Tumor Markers", *supra*). The composition of the biomarker panel in AVENIO^{®} ctDNA Analysis Kit is shown in Table 1.

**Table 1. Composition of the biomarker panel**

| | | | | | | | |
|---|---|---|---|---|---|---|---|
| *ABCC5* | *CSMD1* | *FAT1* | *HTR1E* | *MAP7D 3* | *PIK3CA* | *SV2A* | *AR* |
| *ABCG2* | *CSMD3* | *FBN2* | *HTR2C* | *MKRN3* | *PIK3CG* | *T* | *CCND1* |
| *ACTN2* | *CTNNB 1* | *FBXL7* | *IFI16* | *MMP16* | *PKHD1L1* | *THSD7A* | *CCND2* |
| *ADAMT S12* | *CTNND 2* | *FBXW 7* | *IL7R* | *MTX1* | *POLE* | *TIAM1* | *CCND3* |
| *ADAMT S16* | *CYBB* | *FCRL5* | *INSL3* | *MYH7* | *POMI21L 12* | *TMEM2 00A* | *CD274* |
| *ARFGEF 1* | *DCAF1 2L1* | *FOXG 1* | *ITGA1 0* | *MYTIL* | *PREX1* | *TNFRSF 21* | *CDK4* |
| *ASTN1* | *DCAF1 2L2* | *FRYL* | *ITSN1* | *NAV3* | *PTPLA* | *TNN* | *CDKN2 A* |
| *ASTN2* | *DCAF4 L2* | *GBA3* | *KCNA5* | *NEURO D4* | *RALYL* | *TNR* | *ESR1* |
| *AVPR1A* | *DCLK1* | *GBP7* | *KCNB2* | *NFE2L2* | *RFX5* | *TRHDE* | *FBXW7* |
| *BCHE* | *DCSTA MP* | *GJA8* | *KCNC2* | *NLGN4 X* | *RIN3* | *TRIM58* | *KEAP1* |
| *BP7FB4* | *DDI1* | *GPR13 9* | *KCNJ3* | *NLRP3* | *RNASE3* | *TRPS1* | *MLH1* |
| *C6* | *DLGAP 2* | *GRIA2* | *KCTDS* | *NMUR1* | *ROBO2* | *UGT3A2* | *MSH2* |
| *C6orf118* | *DMD* | *GRIK3* | *KEAP1* | *NOL4* | *SEMA5B* | *USH2A* | *MSH6* |
| *CA10* | *DNTTI P1* | *GRIN2 B* | *KIAA1 211* | *NPAP1* | *SLC18A3* | *USP29* | *NF2* |
| *CACNA1 E* | *DOCK3* | *GRIN3 B* | *KIF17* | *NROB1* | *SLC39A12* | *VPS13B* | *PDCD1L G2* |
| *CDH12* | *DSC3* | *GRM1* | *KIF19* | *NRXN1* | *SLC6A5* | *WBSCR1 7* | *PMS2* |
| *CDH18* | *DSCAM* | *GRMS* | *KLHL3 1* | *NXPH4* | *SLC8A1* | *WIPF1* | *PTEN* |
| *CDH8* | *EGFLA M* | *GRMS* | *KPRP* | *NYAP2* | *SLITRK1* | *WSCD2* | *RB1* |
| *CDH9* | *EPHA5* | *GSX1* | *LPPR4* | *OPRD1* | *SLITRK4* | *ZC3H12 A* | *SMAD4* |
| *CDKN2 A* | *EPHA6* | *HCN1* | *LRFN5* | *P2RY10* | *SLITRKS* | *ZFPM2* | *SMO* |
| *CHRM2* | *EYS* | *HCRT R2* | *LRP1B* | *PAX6* | *SLPI* | *ZIC1* | *STK11* |
| *CNTN5* | *FAM 13 5B* | *HEBP 1* | *LRRC7* | *PCDHI 5* | *SMAD4* | *ZIC4* | *VHL* |
| *CNTNA* P2 | *FAM15 1A* | *HEC W1* | *LRRTM 1* | *PDYN* | *SOX9* | *ZNF521* | *APC* |
| *CPXCR1* | *FAM5B* | *HS3ST 4* | *LRRTM 4* | *PDZRN 3* | *SPTA1* | *ZSCAN1* | *BRCA1* |
| *CPZ* | *FAM5C* | *HS3ST 5* | *LTBP4* | *PGK2* | *ST6GALN AC3* | N/KRAS | *BRCA2* |
| *CRMP1* | *FAM71 B* | *HTR1 A* | *MAP2* | *PHACT R1* | *STK11* | MET | *EGFR* |
| *ALK* | *PDGFR A* | *RAF1* | *JAK3* | *NFE2L2* | *TSC2* | *MTOR* | *PIK3R1* |
| *BRAF* | *RET* | *RNF43* | *KDR* | *NTRK1* | *TSC1* | *MAP2K2* | *PIK3CA* |
| *DPYD* | *ROS1* | *TERT promo ter* | *MAP2K 1* | *PDGFR B* | *KIT* | *UGT1A1* | *PTCH1* |

In some embodiments, the invention further includes a step of improving the biomarker panel based on the results obtained from the clinical samples. In some embodiments, the invention includes the steps of analyzing the correlation between the presence of a biomarker in the cell-free DNA from a statistically significant number of patients and A) RFS, B) TTR (or DFS), and C) OS. The biomarkers showing a predictive correlation are to be included in the panel. The biomarkers not showing a statistically significant predictive correlation are to be excluded from the panel.

In the context of the present invention, the sequence of a biomarker can be determined via any suitable method known in the art. The suitable method would have sufficient accuracy, *e.g.,* sensitivity and specificity to detect rare sequences with a low rate of errors. In some embodiments, the sequencing method includes an error correction step, such as use of molecular barcodes, error stereotyping and other chemical or computation methods of error suppression as described *e.g.,* in see the patent applications "Identification and Use of Circulating Tumor Markers", *supra.* The sequencing method may include a massively parallel sequencing method, including an array based sequencing (Illumina, San Diego, Cal.), an emulsion-based sequencing (ThermoFisher, Waltham, Mass.) an optical measurement based sequencing (Pacific BioSciences, Menlo Park, Cal.) or a nanopore-based sequencing (Roche Sequencing Solutions, Santa Clara, Cal.), or Oxford Nanopore (Oxford, UK).

The sequence data is compared to a reference genome sequence to determine mutations. In some embodiments, the reference sequence is the canonical human genome assembly e.g., HG38. Changes in the nucleic acid sequence compared to the reference genome are added to determine the mutation score. The final mutation score (mutation burden) is determined by dividing the number of mutations by the number of base pairs sequenced. In some embodiments, one or more filters are applied to select mutations to be scored, i.e., for added to the mutation score. In some embodiments, only mutations in the coding regions are scored. In some embodiments, only non-synonymous mutations in the coding regions are scored. In other embodiments, both non-synonymous and synonymous mutations in the coding regions are scored. In yet other embodiments, to enable detection of lower frequency mutation events, mutations in the cancer driver genes are excluded from scoring.

In some embodiments, the mutation burden determined according to the instant invention is assessed as high or low. In some embodiments, the mutation burden is assessed at the population level wherein the relevant population consists of cancer patients diagnosed with the same type of cancer. In some embodiments, the mutation burden is defined as low if it falls under a quantile in the population and the mutation burden is defined as high if it falls at or above the quantile in the population. The quantile may be a quartile, a fertile or a median. In some embodiments, the quantile is a tertile and the mutation burden is defined as low if it falls under the first tertile in the population and the mutation burden is defined as high if it falls at or above the first tertile in the population.

In some embodiments, the invention also includes a step of assessing the status of a cancer in a patient. In some embodiments, the assessing includes identifying the patient as likely or not likely to experience recurrence of the cancer. In other embodiments, the assessing includes determining prognosis of a patient expressed as predicted duration of progression free survival (PFS) and overall survival (OS). The assessing is based on the mutation burden determined as described herein.

In some embodiments, the invention includes a step of statistical analysis to determine a clinically relevant prediction for a patient. In some embodiments, the prediction is selected from Recurrence Free Survival (RFS), Total Time to Recurrence (TTR) and Overall Survival (OS).

Some embodiments not part of the invention include a step of recommending or administering therapy to a cancer in a patient.

Some embodiments not part of the invention include a step of determining whether the patient is likely to respond to therapy.

In some embodiments, the therapy is chemotherapy, chemoradiation therapy or immunotherapy.

In some embodiments, the therapy is immunotherapy including immune checkpoint modulating therapy with immune checkpoint inhibitors (e.g., antibodies against PD-1, PD-L1, CTLA-4, and LAG-3). It has previously been reported that the overall number and rate of mutation and therefore of potential neoepitopes in cancer cells can be predictive of clinical response to immunotherapy, and particularly to immune checkpoint modulator therapy. (WO2016081947). The mutation estimate according to the present invention allows assessing tumor responsiveness to therapy particularly to immunotherapy such as immune checkpoint modulator therapy. In some embodiments, such therapy involves blockade of programmed cell death 1 (PD-1) using a composition comprising an anti-PD-1 antibody. In some particular embodiments, such therapy involves administration of one or more of nivolumab (BMS-936558), pembrolizumab (MK-3475). In some embodiments, the therapy involves administration of an antibody against the PD-1 ligand PD-L1 including atezolizumab (MPDL3280A), avelumab (MSB0010718C) or durvalumab (MEDI4736). In some embodiments, the therapy involves administration of an antibody against CTLA-4 including ipilimimab and tremelimumab. It has been demonstrated that for certain cancers, including small cell or non-small-cell carcinoma of the lung, bladder cancer, renal carcinoma, head and neck cancers, and melanoma patients with high numbers of mutations are more likely to benefit from treatment with immune checkpoint modulators than those patients with lower mutation loads. In some embodiments, patients with higher numbers of somatic mutations respond better to PD-1 blockade (e.g., with anti-PD-1 or anti-PD-L1 antibodies) than those patients with significantly lower overall mutations. (see WO2016081947).

Somatic mutations such as the ones detected by the method disclosed herein comprise DNA alterations in somatic cells including cancer cells. These mutations are detected in the DAN released by the tumor cells into the blood stream (circulating tumor DNA or ctDNA). Somatic mutations in protein-coding regions can result in new epitopes recognized by the immune system (neoantigens). Upon lifting of the inhibition by PD-1 (e.g., by PD-1 or PD-L1 blocking agents listed above), the tumor comprising neoantigens is subject to attack by the patient's immune system.

Some embodiments not part of the invention provide methods for identifying cancer patients that are likely to respond favorably to treatment with an immune checkpoint modulator and treating the patient with an immune checkpoint modulator. This specification also provides methods not part of the invention for identifying cancer patients that are not likely to respond favorably to treatment with an immune checkpoint modulator and recommending that the patients not be treated with an immune checkpoint modulator. Specifically, an exemplary method not part of the invention is a method of treatment of a cancer patient comprising the steps of isolating nucleic acids from a cell-free blood sample obtained from the patient, in the isolated nucleic acid, determining the sequence of at least a portion of each of the biomarkers listed in Table 1, comparing the sequence to the reference sequence and identifying mutations, determining mutation burden as a ratio of the number of mutations identified and the number of bases of nucleic acid sequenced and administering an immunotherapy agent if the mutation burden is high and not administering the immunotherapy agent if the mutation burden is low. In some embodiments, the immunotherapy agent is an anti-PD-1 antibody such as nivolumab administered every 4 weeks at 480 mg or every 2 weeks at 240 mg. In other embodiments, the immunotherapy agent is an anti-CTLA4 antibody such as ipilimumab administered at 10 mg/kg every 3 weeks.

One aspect of the invention includes a system for detecting mutation burden in a patient. The system comprises a processor and a non-transitory computer readable medium coupled to the processor, the medium comprising code executable by the processor for performing a method comprising the steps of analyzing sequencing data on biomarkers from Table 1, performing sequence comparison and mutation detection, error correction, determining a mutation burden as a ratio of the number of mutations identified and the number of bases of nucleic acid sequenced and determining whether the mutation burden in the sample falls above or below a predetermined threshold, e.g., a population-based threshold. In some embodiments, if the mutation burden is at or above the threshold, the system classifies the patient as having high mutation burden and optionally outputs a prognosis or therapy recommendation for high mutation burden. At the same time, if the mutation burden is below the threshold, the system classifies the patient as having low mutation burden and optionally outputs a prognosis or therapy recommendation for low mutation burden.

In some embodiments, the computer readable medium, which may include one or more storages devices, comprises a database including a listing of available therapies depending on mutation burden in the patient. The computer readable medium further comprises a program code having instructions to generate a report listing suitable therapies.

The system may comprise various functional aspects such a server including a processor for processing digital data, a memory coupled to the processor for storing digital data, an input digitizer coupled to the processor for inputting digital data, program code stored in the memory and accessible by the processor, a display device coupled to the processor and memory for displaying information derived from digital data, data networking, and one or more informational databases. The databases may include patient data, patient sample data, clinical data including prior treatment data, a list of therapies and therapeutic agents, patient tracking data and the like.

### Example 1. In silico comparison of whole exome sequencing and selected gene panel to assess TMB

In this example, the panel of genes from the next-generation sequencing based AVENIO^{®} ctDNA Surveillance Kit (Roche Sequencing Solutions, Pleasanton, Cal.) was used to analyze whole exome sequencing (WES) datasets for lung cancer samples from the The Cancer Genome Atlas (TCGA). The in silico analysis compared WES-derived total mutation burden (TMB) and mutation counts derived from *in silico* capture using the AVENIO Surveillance panel (Table 1). TMB was defined as the number of coding SNVs, insertions and deletions per megabase after filtering out known driver mutations, recurrent mutations in tumor suppressor genes and germline mutations as assessed from presence within dbSNP and ExAC. The *in silico* results confirmed that the AVENIO ctDNA surveillance panel can indeed serve as a surrogate for the TMB measured by WES, as illustrated in Figure 1.

### Example 2. Assessing predictive value of mutation burden in patient samples

In this example, the AVENIO^{®} ctDNA Surveillance Kit panel was used to assess mutations in patient samples. Pre-treatment plasma samples were obtained from a prospective, observational study, where 43 late-stage lung adenocarcinoma patients treated with first-line chemo or chemoradiation therapies were initially assessed. Cell-free DNA (ctDNA) from the patients was isolated and analyzed according to the manufacturer's instructions of the AVENIO^{®} ctDNA Surveillance Kit. Synonymous and prevalent driver mutations were filtered out from the detected somatic mutations prior to calculating a mutation count per megabase. Subjects were classified as high mutation count if the filtered somatic mutation count was above the bottom tertile of their cancer type. Progression-free survival (PFS) and overall survival (OS) of patients in the "high" and "low" mutation count groups was assessed (Figure 2). TMB assessed using the AVENIO Surveillance panel is a predictor of PFS and OS in cancer patients.

## Claims

1. A method for determining mutation burden in a lung cancer patient comprising the steps of:
(a) isolating nucleic acids from a cell-free blood sample obtained from the patient;
(b) in the isolated nucleic acid, determining the sequence of at least a portion of each of the biomarkers listed in Table 1;
(c) comparing the sequence determined in step (b) to the reference sequence and identifying mutations;
(d) determining mutation burden as the ratio of the number of mutations identified in step (c) to the number of bases of nucleic acid sequenced in step (b).

2. The method of claim 1, wherein only non-synonymous mutations are used in determining the ratio in step (d).

3. The method of claim 1-2, wherein mutations in cancer driver genes are excluded from determining the ratio in step (d).

4. A method of determining prognosis for a lung cancer patient comprising the steps of:
(a) isolating nucleic acids from a cell-free blood sample obtained from the patient;
(b) in the isolated nucleic acid, determining the sequence of at least a portion of each of the biomarkers listed in Table 1;
(c) comparing the sequence determined in step (b) to the reference sequence and identifying mutations;
(d) determining mutation burden as a ratio of the number of mutations identified in step (c) to the number of bases of nucleic acid sequenced in step (b);
(e) determining prognosis as poor if the mutation burden is high and determining prognosis as good if the mutation burden is low.

5. A system for determining mutation burden in a lung cancer patient, the system comprising a computer designed to implement an algorithm for detecting mutation burden in a sample from a cancer patient, wherein the algorithm analyses sequencing data on biomarkers from Table 1 and contains steps of mutation detection, error correction, determining a mutation burden as a ratio of the number of mutations identified to the number of bases of nucleic acid sequenced and determining whether the mutation burden in the sample falls above or below the bottom tertile of samples from the same cancer type.

## Patentansprüche

1. Verfahren zum Bestimmen der Mutationsbelastung bei einem Lungenkrebspatienten, umfassend die folgenden Schritte:
(a) Isolieren von Nukleinsäuren aus einer zellfreien Blutprobe, die von dem Patienten erhalten wurde;
(b) Bestimmen der Sequenz mindestens eines Teils jedes der in Tabelle 1 aufgelisteten Biomarker in der isolierten Nukleinsäure;
(c) Vergleichen der in Schritt (b) bestimmten Sequenz mit der Referenzsequenz und Identifizieren von Mutationen;
(d) Bestimmen der Mutationsbelastung als das Verhältnis der in Schritt (c) identifizierten Anzahl von Mutationen zu der Anzahl von in Schritt (b) sequenzierten Nukleinsäurebasen.

2. Verfahren nach Anspruch 1, wobei beim Bestimmen des Verhältnisses in Schritt (d) nur nicht-synonyme Mutationen verwendet werden.

3. Verfahren nach Anspruch 1 - 2, wobei Mutationen in Krebstreibergenen vom Bestimmen des Verhältnisses in Schritt (d) ausgeschlossen sind.

4. Verfahren zum Bestimmen der Prognose für einen Lungenkrebspatienten, umfassend die folgenden Schritte:
(a) Isolieren von Nukleinsäuren aus einer zellfreien Blutprobe, die von dem Patienten erhalten wurde;
(b) Bestimmen der Sequenz mindestens eines Teils jedes der in Tabelle 1 aufgelisteten Biomarker in der isolierten Nukleinsäure;
(c) Vergleichen der in Schritt (b) bestimmten Sequenz mit der Referenzsequenz und Identifizieren von Mutationen;
(d) Bestimmen der Mutationsbelastung als ein Verhältnis der in Schritt (c) identifizierten Anzahl von Mutationen zu der Anzahl von in Schritt (b) sequenzierten Nukleinsäurebasen;
(e) Bestimmen, dass die Prognose schlecht ist, wenn die Mutationsbelastung hoch ist, und Bestimmen, dass die Prognose gut ist, wenn die Mutationsbelastung gering ist.

5. System zum Bestimmen der Mutationsbelastung bei einem Lungenkrebspatienten, wobei das System einen Computer umfasst, der so konstruiert ist, dass er einen Algorithmus zum Nachweisen der Mutationsbelastung in einer Probe von einem Krebspatienten implementiert, wobei der Algorithmus Sequenzierungsdaten zu Biomarkern aus Tabelle 1 analysiert und Schritte von Mutationsnachweis, Fehlerkorrektur, Bestimmen einer Mutationsbelastung als ein Verhältnis der Anzahl von identifizierten Mutationen zu der Anzahl von sequenzierten Nukleinsäurebasen und Bestimmen, ob die Mutationsbelastung in der Probe über oder unter das untere Terzil von Proben von derselben Krebsart fällt, enthält.

## Revendications

1. Procédé de détermination d'une charge de mutation chez un patient atteint d'un cancer du poumon comprenant les étapes de :
(a) isolement d'acides nucléiques d'un échantillon de sang exempt de cellules obtenu auprès du patient ;
(b) dans l'acide nucléique isolé, détermination de la séquence d'au moins une partie de chacun des biomarqueurs répertoriés dans le tableau 1 ;
(c) comparaison de la séquence déterminée à l'étape (b) avec la séquence de référence et identification des mutations ;
(d) détermination de la charge de mutation en tant que rapport du nombre de mutations identifiées à l'étape (c) au nombre de bases d'acide nucléique séquencées à l'étape (b).

2. Procédé selon la revendication 1, dans lequel seules les mutations non synonymes sont utilisées dans la détermination du rapport de l'étape (d).

3. Procédé selon la revendication 1 à 2, dans lequel les mutations des gènes entraînant le cancer sont exclues de la détermination du rapport de l'étape (d).

4. Procédé de détermination du pronostic pour un patient atteint d'un cancer du poumon comprenant les étapes de :
(a) isolement d'acides nucléiques d'un échantillon de sang exempt de cellules obtenu auprès du patient ;
(b) dans l'acide nucléique isolé, détermination de la séquence d'au moins une partie de chacun des biomarqueurs répertoriés dans le tableau 1 ;
(c) comparaison de la séquence déterminée à l'étape (b) avec la séquence de référence et identification des mutations ;
(d) détermination de la charge de mutation en tant que rapport du nombre de mutations identifiées à l'étape (c) au nombre de bases d'acide nucléique séquencées à l'étape (b) ;
(e) détermination du pronostic comme étant mauvais si la charge de mutation est élevée et détermination du pronostic comme étant bon si la charge de mutation est faible.

5. Système de détermination d'une charge de mutation chez un patient atteint d'un cancer du poumon, le système comprenant un ordinateur conçu pour mettre en oeuvre un algorithme de détection d'une charge de mutation dans un échantillon d'un patient atteint d'un cancer, dans lequel l'algorithme analyse les données de séquençage sur les biomarqueurs du tableau 1 et contient des étapes de détection de mutations, correction d'erreurs, détermination d'une charge de mutation en tant que rapport du nombre de mutations identifiées au nombre de bases d'acide nucléique séquencées et détermination si la charge de mutation dans l'échantillon est au-dessus ou au-dessous du tercile inférieur d'échantillons du même type de cancer.
